(19) Europäisches Patentamt
European Patent Office
Office européen des brevets

(11) Numéro de publication : **0 302 787 B1**

(12) **FASCICULE DE BREVET EUROPEEN**

(45) Date de publication du fascicule du brevet : **30.10.91 Bulletin 91/44**

(21) Numéro de dépôt : **88402023.1**

(22) Date de dépôt : **03.08.88**

(51) Int. Cl.[5] : **C07D 491/04**, A61K 31/445, // (C07D491/04, 307:00, 221:00)

# (54) Dérivés de (pipéridinyl-4)méthyl-2 benzofuro[2,3-c]pyridines, leur préparation et leur application en thérapeutique.

(30) Priorité : **07.08.87 FR 8711287**

(43) Date de publication de la demande : **08.02.89 Bulletin 89/06**

(45) Mention de la délivrance du brevet : **30.10.91 Bulletin 91/44**

(84) Etats contractants désignés : **AT BE CH DE ES FR GB GR IT LI LU NL SE**

(56) Documents cités : **EP-A- 0 206 225**

(73) Titulaire : **SYNTHELABO**
**58 rue de la Glacière**
**F-75013 Paris (FR)**

(72) Inventeur : **Sevrin, Mireille**
**73, rue Raymond Losserand**
**F-75014 Paris (FR)**
Inventeur : **George, Pascal**
**39, rue Henri de Vilmorin**
**F-94400 Vitry sur Seine (FR)**
Inventeur : **Morel, Claude**
**25, rue Gabriel Péri**
**Cresly F-78470 Magny les Hameaux (FR)**

(74) Mandataire : **Ludwig, Jacques et al**
**SYNTHELABO, Service Brevets, 22, avenue Galilée, B.P. 72**
**F-92352 Le Plessis Robinson Cédex (FR)**

**EP 0 302 787 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet des dérivés de (pipéridinyl-4)méthyl-2 benzofuro[2,3-c]pyridines, leur préparation et leur application en thérapeutique.

Les composés de l'invention répondent à la formule générale (I)

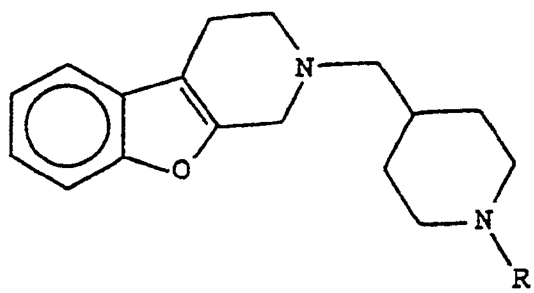

(I)

dans laquelle R représente un groupe benzyle, benzoyle, chloro-3 benzoyle, méthyl-3 benzoyle ou alcoxy($C_1$-$C_6$)carbonyle.

Ils peuvent se présenter à l'état de bases libres ou de sels d'addition à des acides acceptables en pharmacologie.

Conformément à l'invention on peut préparer les composés de formule générale (I) par un procédé illustré par le schéma de la page suivante.

On réduit d'abord la dihydro-3,4 benzofuro[2,3-c]pyridine de formule (II), décrite dans la demande de brevet européen N°0204254, en tétrahydro-1,2,3,4 benzofuro[2,3-c]pyridine de formule (III) au moyen d'un borohydrure alcalin, tel que le borohydrure de sodium, dans un solvant du type alcool aliphatique inférieur tel que le méthanol, à une température de 20 à 40°C.

Puis, si l'on veut préparer le composé de formule générale (I) où R représente un groupe benzyle, on fait réagir le composé de formule (III) avec le chlorure de l'acide benzyl-1 pipéridine-4-carboxylique de formule (IV) (préparé in situ au moyen de l'acide correspondant et de chlorure de thionyle ou de tout autre agent équivalent), dans un solvant inerte tel que le dichlorométhane, en présence d'une base telle que la N,N-diméthylaniline, à une température de 20 à 40°C. Finalement on réduit le composé de formule (V) ainsi obtenu au

Schéma 1

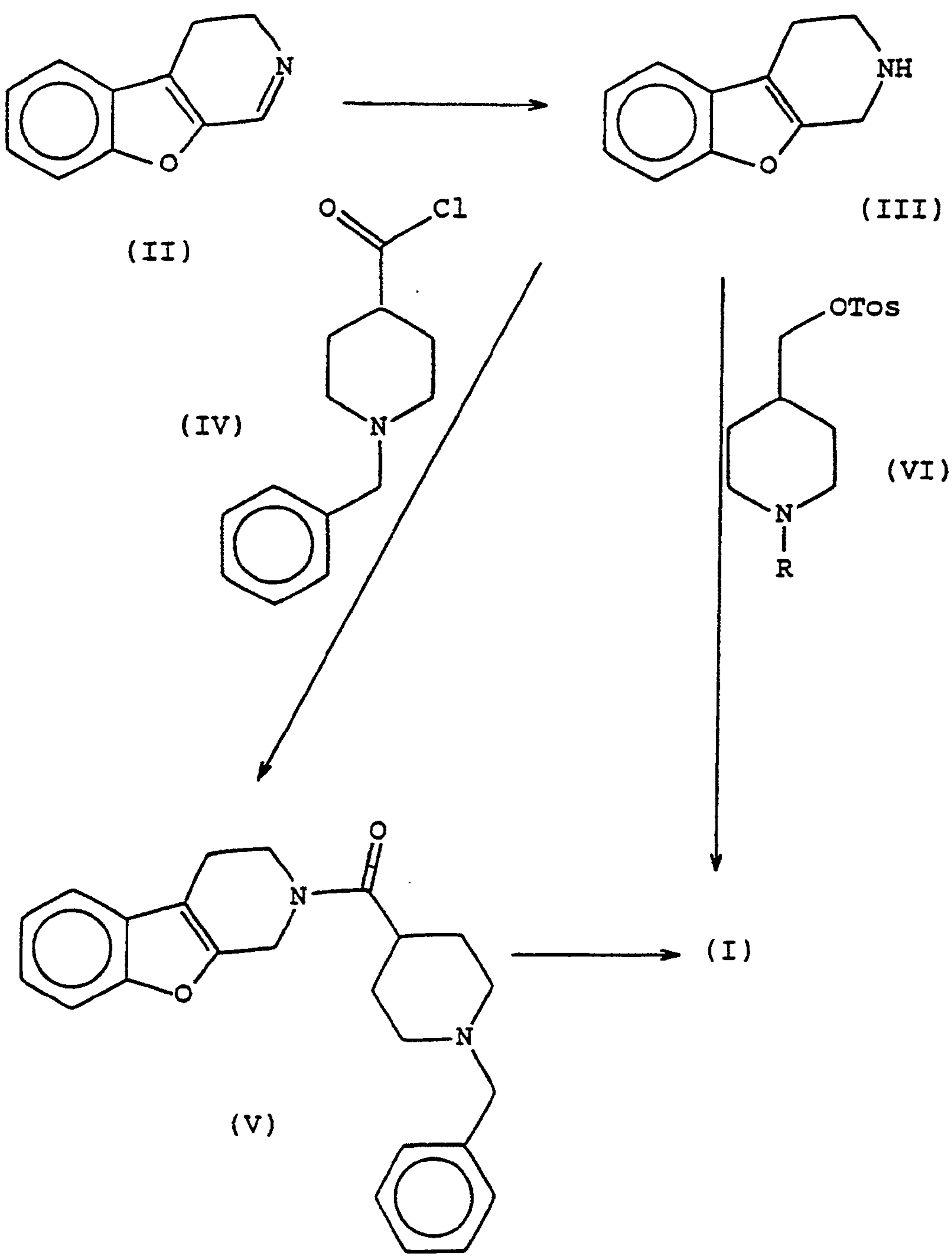

(II) (III) (IV) (VI) (V) (I)

Schéma 2

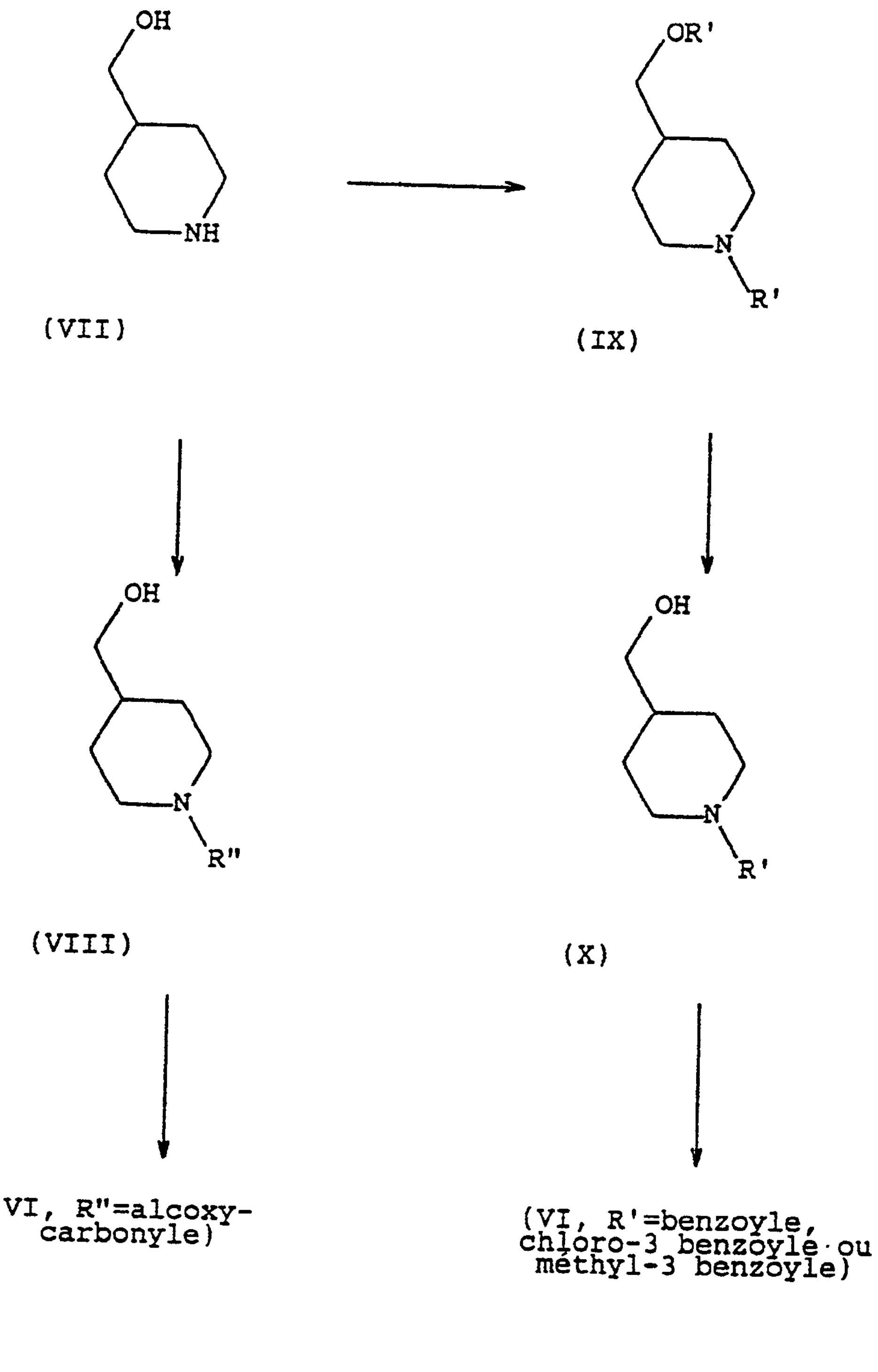

(VII) (IX)

(VIII) (X)

(VI, R"=alcoxy-carbonyle) (VI, R'=benzoyle, chloro-3 benzoyle ou méthyl-3 benzoyle)

moyen d'un hydrure simple ou complexe de bore, tel que le diborane ou le complexe de borane/sulfure de méthyle ou l'hydrure de lithium et d'aluminium, dans un solvant éthéré tel que l'éther diéthylique, le tétrahydrofuranne ou le dioxanne, à une température de 20 à 100°C.

Si l'on veut préparer un composé de formule générale (I) où R représente un groupe benzoyle, chloro-3 benzoyle, méthyl-3 benzoyle ou alcoxy($C_1$-$C_6$)carbonyle, on fait réagir le composé de formule (III) avec un tosylate de formule générale (VI) (dans laquelle Tos représente un groupe tosyle et R représente un groupe benzoyle, chloro-3 benzoyle, méthyl-3 benzoyle ou alcoxy($C_1$-$C_6$)carbonyle), soit en l'absence soit en présence d'un solvant inerte tel que le diméthylformamide ou le xylène, à une température de 20 à 150°C, et éventuellement en présence d'une base organique, telle qu'une amine tertiaire, ou minérale, telle qu'un carbonate ou hydrogénocarbonate alcalin.

Le tosylate de formule générale (VI) peut être préparé selon une méthode illustrée par le schéma 2 qui précède.

Lorsque R représente un groupe benzoyle, chloro-3 benzoyle, ou méthyl-3 benzoyle, on fait réagir le pipéridine-4-méthanol de formule (VII) avec le chlorure d'acide benzoïque, chloro-3 benzoïque, ou méthyl-3 benzoïque, dans un solvant inerte tel qu'un solvant chloré, à une température de 20 à 80°C. On obtient ainsi un ester-amide de formule générale (IX), qu'on saponifie, par exemple au moyen d'hydroxyde de sodium ou de potassium, dans un solvant alcoolique aliphatique inférieur, de préférence l'éthanol, pour obtenir l'alcool de formule générale (X), dont on prépare finalement le tosylate au moyen de chlorure de tosyle, dans un milieu basique tel que la pyridine.

Lorsque R représente un groupe alcoxy($C_1$-$C_6$)carbonyle, on fait réagir le pipéridine-4-méthanol de formule (VII) avec un chloroformiate d'alkyle en $C_1$-$C_6$, dans un solvant tel qu'un solvant chloré, à température ambiante. On obtient ainsi un carbamate de formule générale (VIII) dont on prépare le tosylate comme indiqué précédemment.

Le pipéridine-4-méthanol de formule (VII) peut être obtenu par exemple par réduction de pipéridine-4-carboxylate d'éthyle au moyen d'hydrure de lithium et aluminium, ou encore par une telle réduction de benzyl-1 pipéridine-4-carboxylate d'éthyle suivie d'une hydrogénolyse catalytique sous pression.

Le composé de formule générale (I) où R représente un groupe benzyle peut évidemment être aussi obtenu par réduction du composé de formule générale (I) où R représente un groupe benzoyle, dans les conditions décrites à propos de la réduction du composé de formule (V).

Les exemples suivants illustrent la préparation de quelques composés selon l'invention. Les microanalyses et les spectres IR et RMN confirment les structures des composés obtenus. Les numéros donnés entre parenthèses dans les titres des exemples correspondent à ceux du tableau donné plus loin.

Exemple 1 (Composé N°1)

[(Benzyl-1 pipéridinyl-4)méthyl]-2 tétrahydro-1,2,3,4 benzofuro[2,3-c]pyridine, dichlorhydrate.

1.1 Tétrahydro-1,2,3,4 benzofuro[2,3-c]pyridine, chlorhydrate.

A une suspension de 8,2 g (47,8 mmoles) de dihydro-3,4 benzofuro[2,3-c]pyridine dans 350 ml de méthanol on ajoute, par petites portions, et dans un délai de 3 h, 9,2 g (240 mmoles) de borohydrure de sodium. On agite le mélange pendant 20 h à 20°C puis on évapore le solvant sous pression réduite. On lave le résidu à l'eau et on extrait la tétrahydro-1,2,3,4 benzofuro[2,3-c]pyridine au moyen d'acétate d'éthyle. On décante la phase organique, on la sèche sur sulfate de sodium, on l'évapore sous pression réduite et on traite le résidu par un équivalent d'acide chlorhydrique 0,1 N dans l'alcool isopropylique. On isole le chlorhydrate et on le recristallise dans un mélange d'alcool isopropylique et d'éthanol. Point de fusion : 289-291°C.

1.2 [(Benzyl-1 pipéridinyl-4)carbonyl]-2 tétrahydro-1,2,3,4 benzofuro[2,3-c]pyridine, chlorhydrate.

A 12 ml (165 mmoles) de chlorure de thionyle on ajoute 4,4 g (20 mmoles) d'acide benzyl-1 pipéridine-4-carboxylique, et on agite sous atmosphère inerte. On maintient l'agitation pendant 16 h à 20°C, puis on évapore l'excès de chlorure de thionyle sous pression réduite. On reprend le résidu par 25 ml de toluène et on évapore de nouveau sous pression réduite. On dissout le résidu solide dans du dichlorométhane et on ajoute, sous argon, 5,7 ml (45 mmoles) de N,N-diméthylaniline et 3,4 g (20 mmoles) de tétrahydro-1,2,3,4 benzofuro[2,3-c]pyridine (base) en solution dans 12 ml de dichlorométhane. On agite le mélange pendant 2 h à 20°C puis on le verse dans de l'eau. On isole l'insoluble par filtration, on le lave à l'eau et on le met en suspension dans un mélange d'eau et d'acétate d'éthyle. On traite cette suspension avec de l'ammoniaque, on décante la phase organique, on la lave à l'eau et on la sèche. On évapore le solvant sous pression réduite et on sèche l'amide ainsi obtenu sous vide.

On en prépare le chlorhydrate dans l'alcool isopropylique chlorhydrique 0,1 N et on le recristallise dans l'éthanol. Point de fusion : 226-228°C.

1.3 [(Benzyl-1 pipéridinyl-4)méthyl]-2 tétrahydro-1,2,3,4 benzofuro[2,3-c]pyridine, dichlorhydrate.

A une suspension de 0,4 g (10 mmoles) d'hydrure de lithium et d'aluminium dans 120 ml d'éther diéthylique on ajoute, sous agitation et à 20°C, 2,5 g (6,7 mmoles) de [(benzyl-1 pipéridinyl-4)carbonyl]-2 tétrahydro-1,2,3,4

benzofuro[2,3-c] pyridine (base) puis on chauffe le mélange au reflux pendant 4 h. On refroidit le mélange, on l'hydrolyse, on filtre l'insoluble et on évapore le filtrat sous pression réduite. On sèche le résidu sous vide et on recueille 2,3 g de solide. Point de fusion : 107-109°C. On en prépare le dichlorhydrate au moyen d'alcool isopropylique chlorhydrique 0,1 N et on le recristallise dans un mélange de méthanol et d'éthanol. Point de fusion : 285-288°C.

Exemple 2 (Composé N°4)

[[(Méthyl-3 benzoyl)-1 pipéridinyl-4]méthyl]-2 tétrahydro-1,2,3,4 benzo furo[2,3-c]pyridine, benzènesulfonate.

2.1 Pipéridine-4-méthanol.

Dans un ballon tricol de 4 l muni d'un système d'agitation mécanique et d'un réfrigérant on introduit 28,5 g (0,75 mole) d'hydrure de lithium et aluminium et 1,2 l de tétrahydrofuranne. On ajoute à la suspension obtenue 117,9 g (0,75 mole) de pipéridine-4-carboxylate d'éthyle en solution dans 1,2 l de tétrahydrofuranne, et on agite le mélange pendant 6 h à 20°C. On le refroidit à 0°C, puis on l'hydrolyse en ajoutant successivement 22 ml d'eau, 22 ml d'hydroxyde de sodium 1N et 46 ml d'eau. On agite le mélange 30 mn à 20°C, on le filtre, on lave le précipité au tétrahydrofuranne puis à l'éther. On évapore les solvants sous pression réduite, et on obtient 84,4 g d'une huile qu'on utilise telle quelle dans l'étape suivante.

2.2. Méthyl-3 benzoate de [(méthyl-3 benzoyl)-1 pipéridinyl-4]méthyle.

Dans un ballon tricol de 3 l on introduit, sous atmosphère d'argon, 42,25 g (0,367 mole de pipéridine-4-méthanol, 430 ml de dichloro-1,2 éthane, et on ajoute 82 g (0,81 mole) de triéthylamine puis 125,2 g (0,81 mole) de chlorure de méthyl-3 benzoyle. On chauffe le mélange au reflux pendant 4h30, on ajoute encore 8,2 g (0,08 mole) de triéthylamine et 12,5 g (0,08 mole) de chlorure de méthyl-3 benzoyle, et on chauffe le mélange pendant encore 3 h.

On le filtre, on lave les sels au dichloro-1,2 éthane, on évapore le filtrat sous pression réduite, on dissout le résidu dans de l'acétate d'éthyle, on lave la solution avec une solution aqueuse saturée de chlorure de sodium, on évapore le solvant sous pression réduite, et on recristallise le résidu dans un mélange 1/1 d'alcool isopropylique/acétate d'éthyle. On obtient 80 g d'un solide blanc. Point de fusion : 80-83°C.

2.3. (Méthyl-3 benzoyl)-1 pipéridine-4-méthanol.

A une solution de 80 g (0,23 mole) de méthyl-3 benzoate de [(méthyl-3 benzoyl)-1 pipéridinyl-4]méthyle dans 400 ml d'éthanol, on ajoute une solution de 12,76 g (0,23 mole) d'hydroxyde de potassium dans 75 ml d'éthanol et 75 ml d'eau. On agite le mélange à 20°C pendant 3 h, on évapore le solvant sous pression réduite et on extrait la phase aqueuse à l'acétate d'éthyle. On lave la phase organique avec de l'eau puis avec une solution aqueuse saturée de chlorure de sodium, et on la sèche sur sulfate de magnésium. On évapore le solvant sous pression réduite et on obtient 53 g d'alcool qu'on utilise tel quel dans l'étape suivante.

2.4. Méthyl-4 benzènesulfonate de [(méthyl-3 benzoyl)-1 pipéridinyl-4]méthyle.

A une solution de 52 g (0,22 mole) de (méthyl-3 benzoyl)-1 pipéridine-4-méthanol dans 100 ml de pyridine on ajoute 53,3 g (0,28 mole) de chlorure de méthyl-4 benzènesulfonyle dans 60 ml de pyridine. On agite le mélange à 20°C pendant 4 h, puis on le verse dans de la glace. On extrait la phase au dichlorométhane, on lave la phase organique avec une solution aqueuse 5 N d'acide chlorhydrique et on la sèche sur sulfate de magnésium. On évapore les solvants sous pression réduite et on obtient 70 g de solide blanc. Point de fusion: 68-70°C.

2.5 [[(Méthyl-3 benzoyl)-1 pipéridinyl-4]méthyl]-2 tétrahydro-1,2,3,4 benzofuro[2,3-c]pyridine, benzènesulfonate.

Dans un ballon muni d'une agitation magnétique et placé sous argon on introduit 1,1 g (6,3 mmoles de tétrahydro-1,2,3,4 benzofuro[2,3-c]pyridine, 2,7 g (7 mmoles) de méthyl-4 benzènesulfonate de [(méthyl-3 benzoyl)-1 pipéridinyl-4]méthyle, 1,93 g de carbonate de potassium et 10 ml de diméthylformamide. On agite le mélange pendant 5 h à 120°C puis 12 h à 20°C. On verse le mélange sur de la glace, on l'extrait avec de l'acétate d'éthyle, on lave la phase organique à l'eau puis avec une solution aqueuse saturée de chlorure de sodium,

on la sèche sur sulfate de magnésium, on évapore le solvant sous pression réduite et on purifie le résidu par chromatographie sur colonne de silice en éluant avec de l'éther. On obtient ainsi 0,8 g de base.

On en dissout 0,6 g (1,51 mmole) dans 30 ml d'éthanol, on ajoute une solution de 0,239 g (1,51 mmole) d'acide benzènesulfonique, on agite le mélange pendant 30 mn, on évapore le solvant sous pression réduite, on agite le résidu dans de l'acétate d'éthyle, on le filtre et on le sèche. On isole finalement 0,71 g de benzènesulfonate. Point de fusion : 166-169°C.

Exemple 3 (Composé N°5)

[(Tétrahydro-1,2,3,4 benzofuro[2,3-c]pyridinyl-2)méthyl]-4 pipéridine-1-carboxylate d'éthyle, fumarate.

3.1 Benzyl-1 pipéridine-4-méthanol, chlorhydrate.

A une suspension de 18 g (473 mmoles) d'hydrure de lithium et aluminium dans 1 l d'éther on ajoute, sous argon, 117 g (473 mmoles) benzyl-1 pipéridine-4-carboxylate d'éthyle en solution dans 1,5 l d'éther. On agite le mélange à 20°C pendant 1 h puis on l'hydrolyse avec 34 ml d'eau, on le filtre en rinçant le solide à l'éther, et on fait barboter un courant de gaz chlorhydrique dans le filtrat. On obtient 105,4 g de chlorhydrate. Point de fusion : 181,5-185°C.

3.2 Pipéridine-4-méthanol, chlorhydrate.

On introduit dans un flacon de Parr 105 g de chlorhydrate de benzyl-1 pipéridine-4-méthanol, 2 l d'éthanol, 12,5 g de charbon palladié, et on effectue une hydrogénolyse à 50°C sous une pression de 0,40 MPa. On filtre le mélange et on évapore le filtrat sous pression réduite. On obtient 58,7 g de solide. Point de fusion : 128-130°C.

3.3 Hydroxyméthyl-4 pipéridine-1-carboxylate d'éthyle.

A une solution de 61,6 g (406 mmoles) de chlorhydrate de pipéridine-4-méthanol dans un mélange de 406 ml de chloroforme et 406 ml d'eau, on ajoute 168,3 g (1,22 mole) de carbonate de potassium puis 42,55 ml (440 mmoles) de chloroformiate d'éthyle en solution dans 160 ml de chloroforme. On agite le mélange pendant 3 h à 20°C, on sépare la phase organique, on extrait la phase aqueuse au dichlorométhane, on réunit les phases organiques en une seule, on la lave à l'eau, on la sèche sur sulfate de magnésium, et on évapore les solvants sous pression réduite. On obtient 76 g d'une huile qu'on utilise telle quelle dans l'étape suivante.

3.4 [(Méthyl-4 phénylsulfonyloxy)méthyl]-4 pipéridine-1-carboxylate d'éthyle.

A une solution de 71,3 g (380 mmoles) de hydroxyméthyl-4 pipéridine-1-carboxylate d'éthyle dans 57 ml de pyridine on ajoute 86,9 g (456 mmoles) de chlorure de méthyl-4 benzènesulfonyle en solution dans 81 ml de pyridine. On agite le mélange pendant 12 h à 20°C, on le verse dans de l'eau glacée, on l'extrait à l'éther, on sépare la phase organique, on la lave à l'eau puis avec une solution diluée d'acide chlorhydrique, on la sèche sur sulfate de magnésium, on évapore le solvant sous pression réduite et on recristallise le résidu dans le cyclohexane. On obtient 123 g de solide blanc. Point de fusion : 65-66°C.

3.5 [(Tétrahydro-1,2,3,4 benzofuro[2,3-c]pyridinyl-2)-méthyl]-4 pipéridine-1-carboxylate d'éthyle, fumarate.

A une solution de 1,7 g (10 mmoles) de tétrahydro-1,2,3,4 benzofuro[2,3-c]pyridine (base) en solution dans 30 ml de xylène on ajoute 2,8 g (20 mmoles) de carbonate de potassium et 3,4 g (10 mmoles) de [(méthyl-4 phénylsulfonyloxy)méthyl]-4 pipéridine-1-carboxylate d'éthyle. On chauffe la suspension pendant 26 h à 140°C puis on la refroidit. On filtre l'insoluble, on concentre le filtrat sous pression réduite et on purifie le résidu par chromatographie sur colonne de silice. On en prépare le fumarate dans l'éthanol et on le recristallise dans l'acétone. On obtient ainsi 3,4 g de solide jaune. Point de fusion : 167-170°C.

Le tableau ci-après illustre les structures chimiques et les propriétés physiques de quelques composés selon l'invention.

Tableau

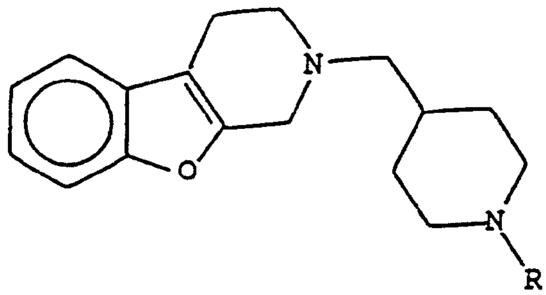

(I)

| N° | R | Sel | F(°C) |
|---|---|---|---|
| 1 | $C_6H_5-CH_2-$ | dichlorhydrate | 285-288 |
| 2 | $C_6H_5-CO-$ | benzènesulfonate | 203-204 |
| 3 | $3-Cl-C_6H_4-CO-$ | benzènesulfonate | 166-168 |
| 4 | $3-CH_3-C_6H_4-CO-$ | benzènesulfonate hémihydraté | 166-169 |
| 5 | $C_2H_5-O-CO-$ | fumarate | 167-170 |

Les composés de l'invention ont été soumis à une série d'essais pharmacologiques qui ont mis en évidence leur intérêt comme substances à activités thérapeutiques.

Ainsi ils ont fait l'objet d'une étude quant à leur affinité pour les récepteurs sérotoninergiques du type 5-$HT_{1A}$.

Les composés déplacent dans l'hippocampe du rat un ligand spécifique marqué, la [$^3$H]-hydroxy-8 dipropylamino-2 tétraline (désignée ci-après par "[$^3$H]-8-OH-DPAT") décrite par Gozlan et coll., Nature, (1983), 305, 140-142.

Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés trois fois à 4°C, en les centrifugeant à chaque fois à 48000 xg et en remettant le culot en suspension pendant 10 mn dans du tampon frais refroidi. Finalement on met le dernier culot en suspension dans le tampon pour arriver à une concentration

de 100 mg de tissu de départ par ml de tampon à 50 mM.

On laisse ensuite incuber à 37°C pendant 10 mn.

La liaison avec la [$^3$H]-8-OH-DPAT est déterminée par incubation de 10 µl de suspension de membranes dans un volume final de 1 ml de tampon contenant 10 µM de pargylline.

Après l'incubation on récupère les membranes par filtration sur filtres Whatman GF/B qu'on lave trois fois avec des quantités aliquotes de 5 ml de tampon glacé. On extrait les filtres dans le liquide de scintillation et on en mesure la radioactivité par scintigraphie liquide. La liaison spécifique de la [$^3$H]-8-OH-DPAT est définie comme la quantité radioactive retenue sur les filtres et pouvant être inhibée par co-incubation dans la hydroxy-5 tryptamine à 10 µM. A une concentration de 1 nM de [$^3$H]-8-OH-DPAT la liaison spécifique représente de 70 à 80% de la radioactivité totale recupérée sur le filtre.

Pour chaque concentration de composés étudié on détermine le pourcentage d'inhibition de la liaison avec la [$^3$H]-8-OH- DPAT, puis la concentration $CI_{50}$, concentration qui inhibe 50% de la liaison.

Pour les composés de l'invention les $CI_{50}$ se situent entre 0,001 et 0,3 µM.

L'activité centrale des composés de l'invention a été évaluée par leurs effets sur les "pointes PGO" (ponto-géniculo-occipitales) induites par la réserpine (test PGO-R) chez le chat, selon la méthode décrite par H. Depoortere, Sleep 1976, 3rd Europ. Congr. Sleep Res., Montpellier 1976, 358-361 (Karger, Basel 1977).

On administre des doses cumulatives de composés à étudier (de 0,01 à 3 mg/kg par voie intraveineuse) à des intervalles de temps de 30 mn, 4 h après injection intrapéritonéale d'une dose de 0,75 mg/kg de réserpine, à des chats curarisés, sous ventilation artificielle. On recueille les activités électroencéphalographique et phasique (pointes PGO-R) à l'aide d'électrodes corticales et profondes (genouillé latéral). Pour chaque dose de composé étudié on détermine le pourcentage de diminution du nombre de pointes PGO, puis la $DA_{50}$, dose active qui diminue de 50% ce nombre de pointes. Pour les composés de l'invention les $DE_{50}$ se situent entre 0,01 et 1 mg/kg par la voie intraveineuse.

Les résultats des essais montrent que les composés de formule générale (I) possèdent une grande affinité et une grande sélectivité pour les récepteurs sérotoninergiques de type 5-$HT_{1A}$. In vivo ils montrent une activité soit agoniste, soit agoniste partielle, soit antagoniste au niveau de ces récepteurs.

Les composés de l'invention peuvent donc être utilisés pour le traitement des maladies et affections impliquant les récepteurs sérotoninergiques de type 5-$HT_{1A}$ de façon directe ou indirecte, notamment pour le traitement des états dépressifs, des états d'anxiété, des troubles du sommeil, dans la régulation de la prise de nourriture, et également pour le traitement de désordres vasculaires, cérébrovasculaires ou cardiovasculaires tels que la migraine ou l'hypertension.

A cet effet ils peuvent être présentés sous toutes formes appropriées à leur administration par voie orale ou parentérale, associés à tous excipients convenables, et dosés pour permettre une posologie journalière de 1 à 1000 mg.

## Revendications

### Revendications pour les Etats contractants suivants : AT, BE, CH, DE, FR, GB, IT, LI, LU, NL, SE

1. Composés de formule générale (I)

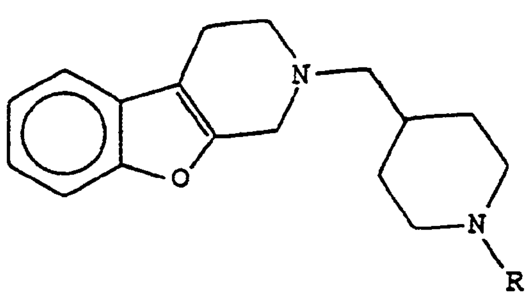

(I)

dans laquelle R représente un groupe benzyle, benzoyle, chloro-3 benzoyle, méthyl-3 benzoyle ou alcoxy($C_1$-$C_6$)carbonyle, ainsi que leurs sels d'addition à des acides acceptables en pharmacologie.

2. Procédé de préparation des composés selon la revendication 1, caractérisé en ce qu'on réduit d'abord la dihydro-3,4 benzofuro[2,3-c]pyridine en tétrahydro-1,2,3,4 benzofuro[2,3-c]pyridine au moyen d'un borohydrure alcalin, tel que le borohydrure de sodium, dans un solvant du type alcool aliphatique inférieur tel que le méthanol, à une température de 20 à 40°C, puis

— pour les composés de formule générale (I) où R représente un groupe benzyle,

on fait réagir la tétrahydro-1,2,3,4 benzofuro[2,3-c]pyridine avec le chlorure de l'acide benzyl-1 pipéridine-4-carboxylique (préparé in situ au moyen de l'acide correspondant et de chlorure de thionyle ou de tout autre agent équivalent), dans un solvant inerte tel que le dichlorométhane, en présence d'une base telle que la N,N-diméthylaniline, à une température de 20 à 40°C, puis on réduit la [(benzyl-1 pipéridinyl-4)carbonyl]-2 tétrahydro-1,2,3,4 benzofuro[2,3-c]pyridine ainsi obtenue au moyen d'un hydrure simple ou complexe de bore, tel que le diborane ou le complexe de borane/sulfure de méthyle, ou d'hydrure de lithium et d'aluminium, dans un solvant éthéré tel que l'éther diéthylique, le tétrahydrofuranne ou le dioxanne, à une température de 20 à 100°C, ou bien

— pour les composés de formule générale (I) où R représente un groupe benzoyle, chloro-3 benzoyle, méthyl-3 benzoyle ou alcoxy($C_1$-$C_6$)carbonyle

on fait réagir la tétrahydro-1,2,3,4 benzofuro[2,3-c]pyridine avec un tosylate de formule générale (VI)

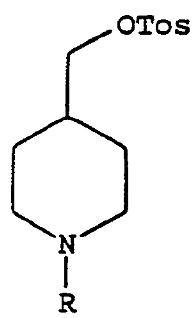

(VI)

(dans laquelle Tos représente un groupe tosyle et R représente un groupe benzoyle, chloro-3 benzoyle, méthyl-3 benzoyle ou alcoxy($C_1$-$C_6$)carbonyle), soit en l'absence soit en présence d'un solvant inerte tel que le diméthylformamide ou le xylène, à une température de 20 à 150°C, et éventuellement en présence d'une base, organique telle qu'une amine tertiaire, ou minérale telle qu'un carbonate ou hydrogénocarbonate alcalin.

3. Composition pharmaceutique caractérisée en ce qu'elle contient un composé selon la revendication 1, associé à un excipient approprié.

**Revendication pour les Etats contractants suivants : ES, GR**

1. Procédé de préparation de composés de formule générale (I)

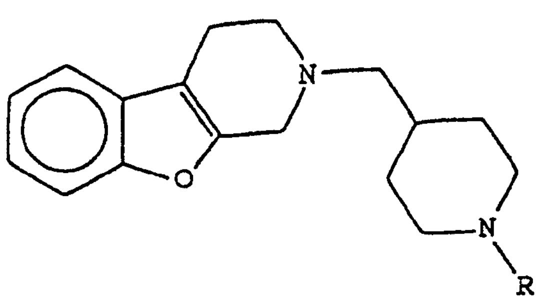

(I)

dans laquelle R représente un groupe benzyle, benzoyle, chloro-3 benzoyle, méthyl-3 benzoyle ou alcoxy($C_1$-$C_6$)carbonyle,

procédé caractérisé en ce qu'on réduit d'abord la dihydro-3,4 benzofuro[2,3-c]pyridine en tétrahydro-1,2,3,4 benzofuro[2,3-c]pyridine au moyen d'un borohydrure alcalin, tel que le borohydrure de sodium, dans un solvant du type alcool aliphatique inférieur tel que le méthanol, à une température de 20 à 40°C, puis

— pour les composés de formule générale (I) où R représente un groupe benzyle,

on fait réagir la tétrahydro-1,2,3,4 benzofuro[2,3-c]pyridine avec le chlorure de l'acide benzyl-1 pipéridine-4-carboxylique (préparé in situ au moyen de l'acide correspondant et de chlorure de thionyle ou de tout autre agent équivalent), dans un solvant inerte tel que le dichlorométhane, en présence d'une base telle

que la N,N-diméthylaniline, à une température de 20 à 40°C, puis on réduit la [(benzyl-1 pipéridinyl-4)carbonyl]-2 tétrahydro-1,2,3,4 benzofuro[2,3-c]pyridine ainsi obtenue au moyen d'un hydrure simple ou complexe de bore, tel que le diborane ou le complexe de borane/sulfure de méthyle, ou d'hydrure de lithium et d'aluminium, dans un solvant éthéré tel que l'éther diéthylique, le tétrahydrofuranne ou le dioxanne, à une température de 20 à 100°C, ou bien

— pour les composés de formule générale (I) où R représente un groupe benzoyle, chloro-3 benzoyle, méthyl-3 benzoyle ou alcoxy($C_1$-$C_6$)carbonyle

on fait réagir la tétrahydro-1,2,3,4 benzofuro[2,3-c]pyridine avec un tosylate de formule générale (VI)

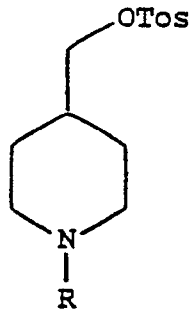

(VI)

(dans laquelle Tos représente un groupe tosyle et R représente un groupe benzoyle, chloro-3 benzoyle, méthyl-3 benzoyle ou alcoxy($C_1$-$C_6$)carbonyle), soit en l'absence soit en présence d'un solvant inerte tel que le diméthylformamide ou le xylène, à une température de 20 à 150°C, et éventuellement en présence d'une base, organique telle qu'une amine tertiaire, ou minérale telle qu'un carbonate ou hydrogénocarbonate alcalin.

## Patentansprüche

### Patentansprüche für folgende Vertragsstaaten : AT, BE, CH, DE, FR, GB, IT, LI, LU, NL, SE

1. Verbindungen der allgemeinen Formel (I)

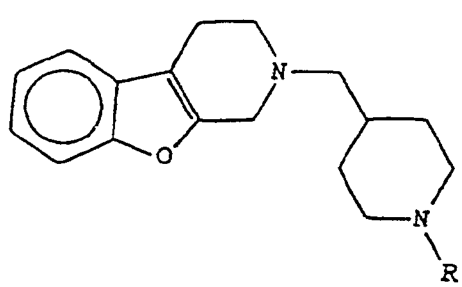

(I)

in welcher R eine Benzyl-, Benzoyl-, 3-Chlorbenzoyl-, 3-Methylbenzoyl- oder ($C_1$-$C_6$)-Alkoxycarbonylgruppe darstellt, sowie deren Additionssalze mit pharmakologisch verwendbaren Säuren.

2. Verfahren zur Herstellung von Verbindungen nach Anspruch 1, dadurch gekennzeichnet, daß man zuerst 3,4-Dihydrobenzofuro[2,3-c]pyridin mit Hilfe eines Alkaliborhydrids, wie Natriumborhydrid, in einem Lösungsmittel von der Art niedriger aliphatischer Alkohole, wie Methanol, bei einer Temperatur von 20 bis 40°C zu 1,2,3,4-Tetrahydrobenzofuro[2,3-c]pyridin reduziert, dann

— für die Verbindungen der allgemeinen Formel (I), in welcher R für eine Benzylgruppe steht,

das 1,2,3,4-Tetrahydrobenzofuro[2,3-c]pyridin in einem inerten Lösungsmittel, wie Dichlormethan, in Gegenwart einer Base, wie N,N-Dimethylanilin, bei einer Temperatur von 20 bis 40°C mit 1-Benzylpiperidin-4-carbonsäurechlorid (in situ hergestellt aus der entsprechenden Säure mit Thionylchlorid oder einem anderen äquivalenten Mittel) reagieren läßt, dann das so erhaltene 2-(1-Benzylpiperidinyl-4-carbonyl)-1,2,3,4-tetrahydrobenzofuro[2,3-c]pyridin mit einem einfachen Borhydrid oder Borkomplex, wie dem Diboran oder dem Boran/Methylsulfid-Komplex, oder mit Lithiumaluminiumhydrid in einem etherischen Lösungsmittel, wie Diethylether, Tetrahydrofuran oder Dioxan, bei einer Temperatur von 20 bis 100°C reduziert oder aber

— für die Verbindungen der allgemeinen Formel (I), in welcher R für eine Benzoyl-, 3-Chlorbenzoyl-, 3-Methylbenzoyl- oder ($C_1$-$C_6$)-Alkoxycarbonylgruppe steht,

das 1,2,3,4-Tetrahydrobenzofuro[2,3-c]pyridin mit einem Tosylat der allgemeinen Formel (VI)

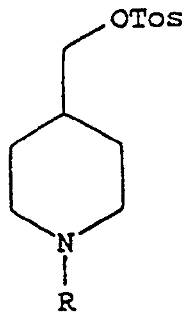

(VI)

(in welcher Tos für eine Tosylgruppe und R für eine Benzoyl-, 3-Chlorbenzoyl-, 3-Methylbenzoyl oder ($C_1$-$C_6$)-Alkoxycarbonylgruppe steht) in An- oder Abwesenheit eines inerten Lösungsmittels, wie Dimethylformamid oder Xylol, bei einer Temperatur von 20 bis 150°C und gegebenenfalls in Gegenwart einer organischen Base, wie eines tertiären Amins, oder einer mineralischen Base, wie eines Alkalicarbonates oder -hydrogencarbonates, reagieren läßt.

3. Pharmazeutische Zusammensetzung, **dadurch gekennzeichnet**, daß sie eine Verbindung nach Anspruch 1 in Kombination mit einem geeigneten Bindemittel enthält.

**Patentanspruch für folgenden Vertragsstaaten : ES, GR**

1. Verfahren zur Herstellung von Verbindungen der allgemeinen Formel (I)

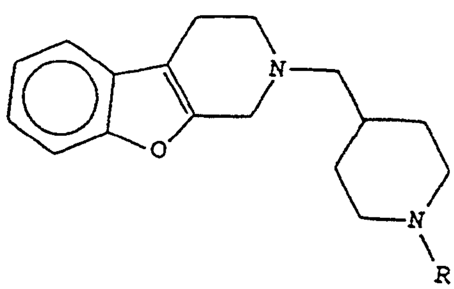

(I)

in welcher R eine Benzyl-, Benzoyl-, 3-Chlorbenzoyl-, 3-Methylbenzoyl- oder ($C_1$-$C_6$)-Alkoxycarbonylgruppe darstellt, **dadurch gekennzeichnet**, daß man zuerst 3,4-Dihydrobenzofuro[2,3-c]pyridin mit Hilfe eines Alkaliborhydrids, wie Natriumborhydrid, in einem Lösungsmittel von der Art niedriger aliphatischer Alkohole, wie Methanol, bei einer Temperatur von 20 bis 40°C zu 1,2,3,4-Tetrahydrobenzofuro[2,3-c]pyridin reduziert, dann

— für die Verbindungen der allgemeinen Formel (I), in welcher R für eine Benzylgruppe steht,

das 1,2,3,4-Tetrahydrobenzofuro[2,3-c]pyridin in einem inerten Lösungsmittel, wie Dichlormethan, in Gegenwart einer Base, wie N,N-Dimethylanilin, bei einer Temperatur von 20 bis 40°C mit 1-Benzylpiperidin-4-carhonsäurechlorid (in situ hergestellt aus der entsprechenden Säure mit Thionylchlorid oder einem anderen äquivalenten Mittel) reagieren läßt, dann das so erhaltene 2-(1-Benzylpiperidinyl-4-carbonyl)-1,2,3,4-tetrahydrobenzofuro[2,3-c]pyridin mit einem einfachen Borhydrid oder Borkomplex, wie dem Diboran oder dem Boran/Methylsulfid-Komplex, oder mit Lithiumaluminiumhydrid in einem etherischen Lösungsmittel, wie Diethylether, Tetrahydrofuran oder Dioxan, bei einer Temperatur von 20 bis 100°C reduziert oder aber

— für die Verbindungen der allgemeinen Formel (I), in welcher R für eine Benzoyl-, 3-Chlorbenzoyl-, 3-Methylbenzoyl- oder ($C_1$-$C_6$)-Alkoxycarbonylgruppe steht,

das 1,2,3,4-Tetrahydrobenzofuro[2,3-c]pyridin mit einem Tosylat der allgemeinen Formel (VI)

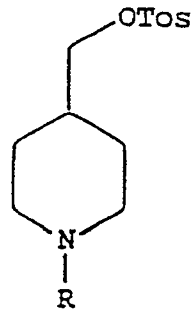

(VI)

(in welcher Tos für eine Tosylgruppe und R für eine Benzoyl-, 3-Chlorbenzoyl-, 3-Methylbenzoyl oder ($C_1$-$C_6$)-Alkoxycarbonylgruppe steht) in An- oder Abwesenheit eines inerten Lösungsmittels, wie Dimethylformamid oder Xylol, bei einer Temperatur von 20 bis 150°C und gegebenenfalls in Gegenwart einer organischen Base, wie eines tertiären Amins, oder einer mineralischen Base, wie eines Alkalicarbonates oder -hydrogencarbonates, reagieren läßt.

## Claims

**Claims for the following Contracting States : AT, BE, CH, DE, FR, GB, IT, LI, LU, NL, SE**

1. Compounds of general formula (I)

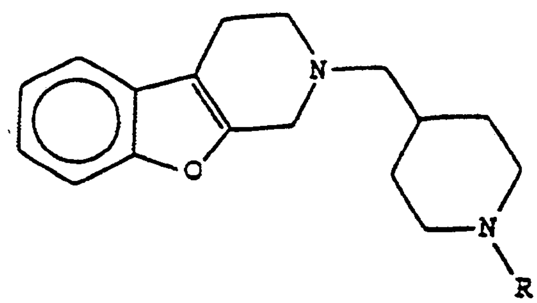

(I)

in which R denotes a benzyl, benzoyl, 3-chlorobenzoyl, 3-methylbenzoyl or ($C_1$-$C_6$ alkoxy)carbonyl group, as well as their addition salts with pharmacologically acceptable acids.

2. Process for preparing the compounds according to Claim 1, characterised in that 3,4-dihydrobenzofuro[2,3-c]pyridine is first reduced to 1,2,3,4-tetrahydrobenzofuro[2,3-c]pyridine by means of an alkali metal borohydride, such as sodium borohydride, in a solvent of the lower aliphatic alcohol type, such as methanol, at a temperature of 20 to 40°C, and then

— for the compounds of general formula (I) in which R denotes a benzyl group,
the 1,2,3,4-tetrahydrobenzofuro[2,3-c]pyridine is reacted with 1-benzyl-4-piperidinecarboxylic acid chloride (prepared in situ by means of the corresponding acid and thionyl chloride or any other equivalent agent), in an inert solvent such as dichloromethane, in the presence of a base such as N,N-dimethylaniline, at a temperature of 20 to 40°C, and the 2-[(1-benzyl-4-piperidyl)carbonyl]-1,2,3,4-tetrahydrobenzofuro[2,3-c]pyridine thereby obtained is then reduced by means of a simple or complex boron hydride, such as diborane or the borane/methyl sulphide complex, or lithium aluminium hydride, in an ethereal solvent such as diethyl ether, tetrahydrofuran or dioxane, at a temperature of 20 to 100°C, or alternatively,

— for the compounds of general formula (I) in which R denotes a benzoyl, 3-chlorobenzoyl, 3-methylbenzoyl or ($C_1$-$C_6$ alkoxy)carbonyl group,
the 1,2,3,4-tetrahydrobenzofuro[2,3-c]pyridine is reacted with a tosylate of general formula (VI)

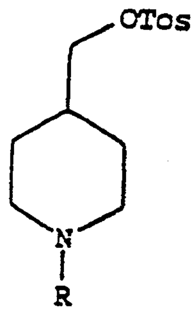

(VI)

(in which Tos denotes a tosyl group and R denotes a benzoyl, 3-chlorobenzoyl, 3-methylbenzoyl or ($C_1$-$C_6$ alkoxy)carbonyl group, either in the absence or in the presence of an inert solvent such as dimethylformamide or xylene, at a temperature of 20 to 150°C, and optionally in the presence of an organic base such as a tertiary amine, or an inorganic base such as an alkali metal carbonate or hydrogen carbonate.

3. Pharmaceutical composition, characterised in that it contains a compound according to Claim 1, in combination with a suitable excipient.

**Claim for the following Contracting States : ES, GR**

1. Process for preparing the compounds of general formula (I)

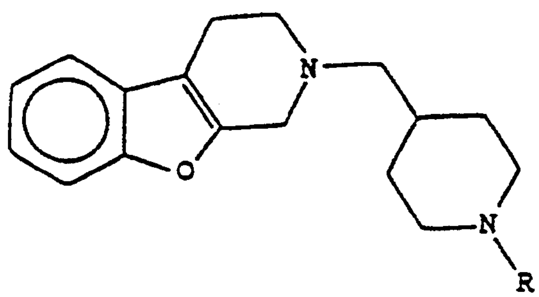

(I)

in which R denotes a benzyl, benzoyl, 3-chlorobenzoyl, 3-methylbenzoyl or ($C_1$-$C_6$ alkoxy)carbonyl group, which process is characterised in that 3,4-dihydrobenzofuro-[2,3-c]pyridine is first reduced to 1,2,3,4-tetrahydrobenzofuro[2,3-c]pyridine by means of an alkali metal borohydride, such as sodium borohydride, in a solvent of the lower aliphatic alcohol type, such as methanol, at a temperature of 20 to 40°C, and then

— for the compounds of general formula (I) in which R denotes a benzyl group, the 1,2,3,4-tetrahydrobenzofuro[2,3-c]pyridine is reacted with 1-benzyl-4-piperidinecarboxylic acid chloride (prepared in situ by means of the corresponding acid and thionyl chloride or any other equivalent agent), in an inert solvent such as dichloromethane, in the presence of a base such as N,N-dimethylaniline, at a temperature of 20 to 40°C, and the 2-[(1-benzyl-4-piperidyl)carbonyl]1,2,3,4-tetrahydrobenzofuro[2,3-c]pyridine thereby obtained is then reduced by means of a simple or complex boron hydride, such as diborane or the borane/methyl sulphide complex, or lithium aluminium hydride, in an ethereal solvent such as diethyl ether, tetrahydrofuran or dioxane, at a temperature of 20 to 100°C, or alternatively,

— for the compounds of general formula (I) in which R denotes a benzoyl, 3-chlorobenzoyl, 3-methylbenzoyl or ($C_1$-$C_6$ alkoxy)carbonyl group, the 1,2,3,4-tetrahydrobenzofuro[2,3-c]pyridine is reacted with a tosylate of general formula (VI)

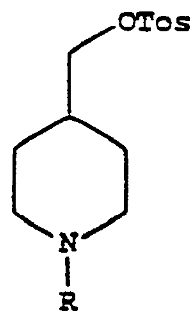

(VI)

(in which Tos denotes a tosyl group and R denotes a benzoyl, 3-chlorobenzoyl, 3-methylbenzoyl or ($C_1$-$C_6$ alkoxy)carbonyl group, either in the absence or in the presence of an inert solvent such as dimethylformamide or xylene, at a temperature of 20 to 150°C, and optionally in the presence of an organic base such as a tertiary amine, or an inorganic base such as an alkali metal carbonate or hydrogen carbonate.